Europäisches Patentamt

European Patent Office

Office européen des brevets

(19)

(11) Numéro de publication: **0 355 245**
**A1**

## (12) DEMANDE DE BREVET EUROPEEN

(21) Numéro de dépôt: 88870139.8

(22) Date de dépôt: 26.08.88

(51) Int. Cl.4: **A61K 31/655**

Le titre de l'invention a été modifié (Directives relatives à l'examen pratiqué à l'OEB, A-III, 7.3)

(43) Date de publication de la demande:
28.02.90 Bulletin 90/09

(84) Etats contractants désignés:
**AT BE CH DE ES FR GB GR IT LI LU NL SE**

(71) Demandeur: **Vandevelde, Michel**
**Avenue de la Forêt de Soignes 346a**
**B-1640 Rhode-Saint-Genèse(BE)**

Demandeur: **Margery, Hélène**
**Avenue de la Forêt de Soignes 346a**
**B-1640 Rhodes-Saint-Genèse(BE)**

(72) Inventeur: **Vandevelde, Michel**
**Avenue de la Forêt de Soignes 346a**
**B-1640 Rhode-Saint-Genèse(BE)**
Inventeur: **Margery, Hélène**
**Avenue de la Forêt de Soignes 346a**
**B-1640 Rhodes-Saint-Genèse(BE)**

(74) Mandataire:.Claeys, Pierre et al
**Bureau Gevers rue de Livourne 7 bte 1**
**B-1050 Bruxelles(BE)**

(54) Utilisation d'agents d'inhibition de la réverse-transcriptase des rétrovirus.

(57) Utilisation d'au moins un agent d'inhibition de la réverse-transcriptase des virus du groupe rétrovirus, en particulier de ceux responsables du sida, tels que, entre autres, HTLV I, HTLV II, HTLV III/LAV, pour sa ou leur mise en oeuvre dans ou sur tout produit susceptible d'entrer localement en contact avec la peau, les muqueuses ou des sécrétions corporelles et produits susceptibles d'entrer localement en contact avec la peau, les muqueuses ou des sécrétions corporelles comprenant un tel agent d'inhibition.

EP 0 355 245 A1

## Utilisation d'agent d'inhibition de la réverse-transcriptase des rétrovirus, et produit susceptible d'entrer en contact avec la peau, les muqueuses ou des sécrétions corporelles.

La présente invention concerne une utilisation d'au moins un agent d'inhibition de la réverse-transcriptase des virus du groupe rétrovirus, en particulier de ceux responsables du sida, tels que, entre autres, HTLV I, HTLV II, HTLV III/LAV. Elle concerne également les produits susceptibles d'entrer localement en contact avec la peau, les muqueuses ou des sécrétions corporelles.

Depuis la détermination relativement récente de la maladie appelée sida, beaucoup de recherches ont été effectuées sur ce sujet. On a pu établir l'existence d'une série de virus, faisant partie du groupe des rétrovirus et parmi lesquels on peut citer les virus lymphotropiques de cellule T humaine, appelés virus HTLV I et HTLV II, ainsi que le virus HTLV III/LAV, principal responsable du sida.

Il a pu être démontré que ces virus étaient présents, chez les porteurs, dans la plupart des liquides intérieurs, notamment dans le sang, la salive, le sperme, la lymphe, le lait, les larmes, les sécrétions vaginales (v. D. ZAGURY et cons., Science, oct. 1984, vol. 226, n° 4673, p. 449-451; R. ZITTOUN, Syndrome Immuno Déficitaire Acquis, Doins Editeurs, Paris, 1986, p. 183).

Si, jusqu'à présent, il n'a pas pu être possible de mettre au point un vaccin ou un médicament efficace contre le sida, il est devenu petit à petit possible d'émettre quelques interprétations à propos du développement de ces virus. Lors d'une "inoculation muqueuse", c'est-à-dire par une muqueuse du sujet infecté, les virus semblent connaître une phase de réplication pendant laquelle les particules restent dans la sphère de la muqueuse du porteur. Cette phase pourrait se prolonger pendant plusieurs mois. Ce n'est que dans une seconde phase que les virus et/ou ses constituants dissémineront à partir de la muqueuse (R. ZITTOUN, op. cit., p. 184).

La reproduction des rétrovirus est permise par la présence d'une enzyme, la réverse-transcriptase. Il est connu que la suramine est un inhibiteur de la réverse-transcriptase d'un certain nombre de rétrovirus. Des essais in vitro ont par ailleurs montré l'efficacité de certaines molécules, notamment de composés disazoïques, tels que la suramine, dans l'inhibition de la réverse-transcriptase du virus HTLV-III (H. MITSUYA et cons., Science, octobre 1984, Vol. 226, n° 4671, p. 172 à 174).

Des essais ultérieurs ont été effectués in vivo sur des porteurs du virus du sida. On leur a administré par voie orale des doses d'inhibiteurs de la réverse-transcriptase, et notamment de suramine. Si le virus ne pouvait plus être décelé, pendant le traitement systémique, dans des prélèvements effectués sur des patients infectés, il s'est manifesté à nouveau peu après la cessation du traitement (S. BRODER et cons., The Lancet, septembre 21, 1985, p. 627 à 630; T.R. HARRISSON, Principes de médecine interne, 4ème éd. française, Paris, 1988, p. 1395). Cette voie semble donc actuellement abandonnée.

La présente invention a pour but de proposer des moyens prophylactiques dans la lutte contre le sida. Au contraire de la tendance actuelle des spécialistes, décrite ci-dessus, elle propose de mettre en valeur, d'une part, la connaissance de l'activité inhibitrice, in vitro, de composés disazoïques sur la réverse-transcriptase du HTLV-III/LVA et, d'autre part, l'interprétation actuelle d'un développement en deux phases du virus lors d'une "infection muqueuse" ou plutôt par contact. L'invention a pour objet de rendre défavorables à la transmission, à la propagation et à la reproduction du virus les produits, qui sont susceptibles, pour une raison quelconque, d'entrer en contact avec la peau, les muqueuses ou des sécrétions corporelles. Il s'agit donc d'une mesure préventive, antérieure à toute application sur le corps humain ou animal.

Pour résoudre ce problème, l'invention prévoit l'utilisation d'au moins un agent d'inhibition de la réverse-transcriptase des virus du groupe rétrovirus, en particulier de ceux responsables du sida, tels que, entre autres, HTLV I, HTLV II, HTLV III/LAV, pour sa ou leur mise en oeuvre dans ou sur tout produit susceptible d'entrer localement en contact avec la peau, les muqueuses ou des sécrétions corporelles.

Suivant un mode de réalisation de l'invention, au moins un des agents d'inhibition est un composé disazoïque. De préférence, un agent d'inhibition est un sel hexasodique d'acide 8,8'-[carbonyl-bis[imino3,1-phénylènecarbonylimino(4-méthyl-3,1-phénylène)-carbonyl-imino]]-bis-1,3,5-naphtalènetrisulfonique. Cette molécule est appelée également suramine sodique, germanine, 205 Bayer, 309 Fourneau, moranyl, antrypol, naganol ou naganine. Cette molécule est connue depuis longtemps, notamment pour le traitement de la trypanosomiase ou de l'onchocercose. Comme on l'a dit précédemment, sa valeur comme agent d'inhibition de la réverse-transcriptase de rétrovirus était connue depuis longtemps et on vient de montrer son activité in vitro contre la réverse-transcriptase du HTLV III/LVA. Cette substance, qui est un dérivé de la benzidine, se présente sous la forme d'une poudre blanche, aisément soluble dans l'eau, et sa toxicité est peu élevée.

On envisage également suivant l'invention, comme autres composés disazoïques, entre autres la benzidine, la dianilidurée, le pyridium, la néotropine, le rouge congo, le bleu de trypan, le rouge de trypan, le violet de trypan.

L'invention prévoit également des produits susceptibles d'entrer localement en contact avec la peau, les muqueuses ou des sécrétions corporelles, ces produits comprenant au moins un agent d'inhibition de la réverse-transcriptase des virus du groupe rétrovirus, en particulier de ceux responsables du sida, tels que HTLV I, HTLV II, HTLV III/LAV, et de préférence de la suramine sodique.

D'autres formes de réalisation avantageuses de l'invention ressortent notamment des sous-revendications 6 à 16.

Les produits suivant l'invention peuvent donc être des supports solides, par exemple des supports fibreux comme des mouchoirs, des serviettes, des bandages, des pansements, de l'ouate, ou des supports en forme de film, à base de matière plastique ou caoutchouteuse, comme des gants de médecin ou de dentiste, des préservatifs ou diaphragmes, des produits d'emballage, etc.

Les produits suivant l'invention forment donc un milieu défavorable à la transmission du virus à une personne saine, en réalisant une espèce de barrière protectrice.

Les produits suivant l'invention peuvent aussi comporter un excipient pâteux ou un véhicule liquide. Par la présence d'au moins un agent suivant l'invention dans les produits cosmétiques ou d'hygiène journalière, comme les dentifrices, ces produits sont, préalablement à leur application, rendus incapables de transmettre le virus, précisément là où l'infection par celui-ci peut se propager aisément. Secondairement, ils peuvent avoir un effet thérapeutique local sur des virus éventuellement présents à l'endroit de l'application du produit. On peut même prévoir aussi un traitement préalable comparable de produits thérapeutiques quelconques, à application locale, par un agent inhibiteur de la réverse-transcriptase du HTLV III/LVA.

L'invention va à présent être illustrée à l'aide de quelques exemples de réalisation non limitatifs.

**Exemple 1**

A 100g d'une pâte dentifrice comprenant 4 % de ricinilate, on mélange 10 mg de suramine sodique.

**Exemple 2**

| Composition d'un bain de bouche : | |
| --- | --- |
| perborate de sodium | 8 g |
| borax | 32 g |
| chlorure de sodium | 20 g |
| bicarbonate de sodium | 40 g |
| suramine sodique | 2 g |
| essence de menthe | 3 gouttes |
| 1 cuillère à café dans une tasse d'eau tiède. | |

**Exemple 3**

| Produit à pulvériser dans la bouche : | |
| --- | --- |
| suramine sodique | 100 mg. |
| alcoolature de citron PBV | 1 goutte |
| eau ad | 10 ml |
| flacon nébulisateur. | |

**Exemple 4**

| Savon solide : | |
|---|---|
| suramine sodique | 1 g |
| savon potassique | 74 g |
| pour 1 brique. | |

## Example 5

| Savon liquide : | |
|---|---|
| suramine sodique | 1,5 g |
| savon potassique | 60 g |
| essence de lavande | 10 gouttes |
| solution antiseptique ad | , 100 g |
| pour 100 g de savon. | |

## Exemple 6

| Shampoing : | |
|---|---|
| dianilidurée | 20 g |
| huile de ricin | 2 g |
| lécithine | 2 g |
| acide undécylique | 4 g |
| Tensomel G 211 | 4 g |
| propylèneglycol | 20 g |
| isopropanol | 20 g |
| Tensergex SP DL 6 | 100 g |
| eau ad | 200 ml |
| pour 200 ml de shampooing. | |

On dissout la lécithine et l'acide undécylique dans le mélange d'isopropanol et d'huile de ricin, puis on ajoute le propylèneglycol et les autres composants. Le produit est à agiter avant l'emploi.

## Exemple 7

| Lotion capillaire : | |
|---|---|
| suramine sodique | 1,5 g |
| nicotinate de benzyle | 25 mg |
| dexpanthoténol | 3 g |
| Teinture de jaborandi | 5 g |
| éthanol | 50 g |
| essence de lavande | 1 g |
| Eau ad | 150 ml |
| pour 150 ml de lotion. | |

Exemple 8

| Lotion après rasage : | |
| --- | --- |
| suramine sodique | 1 g |
| chlorhydrate de diphénydramine | 1 g |
| oxyde de zinc | 8 g |
| liniment au veegum suivant FMS ad | 100 ml |
| pour 100 ml de lotion. | |

Exemple 9

| Crème : | |
| --- | --- |
| suramine sodique | 1 g |
| triéthanolamine | 1 g |
| glycérol | 2,5 g |
| cire blanche | 2,5 g |
| acide stéarique | 6 g |
| huile d'amande | 7,5 g |
| essence de lavande | 2 gouttes |
| aqua conservans ad | 50 g FMS |
| pour 50 g de crème. | |

Exemple 10

| Talc : | |
| --- | --- |
| bleu de trypan | 1,0 g |
| essence de lavande | 5 gouttes |
| talc ad | 100 g |
| pour 100 g de talc. | |

Exemple 11

Condom :

D'une manière courante, un condom en matière caoutchouteuse est lubrifié d'une vaseline renfermant de la suramine sodique :

| suramine sodique | 2 g |
|---|---|
| vaseline ad | 50 g |
| pour 50 g de produit. | |

## Exemple 12

Papier de toilette, bandes et tampons hygiéniques, ouate, etc.

On prépare tout d'abord une poudre à pulvériser dont la composition est par exemple la suivante :

| suramine sodique | 10 g |
|---|---|
| sous-gallate de bismuth | 50 g |
| peroxyde de zinc | 100 g |
| talc | 840 g |
| pour 1 kg de poudre à pulvériser. | |

On pulvérise ensuite d'une manière courante cette poudre qui adhère aux fibres des produits traités.

Il doit être entendu que l'invention n'est en aucune façon limitée aux formes de réalisation décrites ci-dessus et que bien des modifications peuvent y être apportées sans sortir de son cadre.

On peut par exemple envisager que l'agent d'inhibition soit fixé de manière libérable sur un support solide par d'autres moyens que ceux décrits, comme par exemple par imprégnation.

## Revendications

1. Utilisation d'au moins un agent d'inhibition de la réverse-transcriptase des virus du groupe rétrovirus, en particulier de ceux responsables du sida, tels que, entre autres, HTLV I, HTLV II, HTLV III/LAV, pour sa ou leur mise en oeuvre dans ou sur tout produit susceptible d'entrer localement en contact avec la peau, les muqueuses ou des sécrétions corporelles.

2. Utilisation suivant la revendication 1, caractérisée en ce qu'au moins un des agents d'inhibition est un composé disazoïque.

3. Utilisation suivant la revendication 2, caractérisée en ce qu'un agent d'inhibition est un sel hexasodique d'acide 8,8'-[carbonyl-bis-[imino-3,1-phénylènecarbonylimino-(4-méthyl-3,1-phénylène)-carbonyl-imino]]-bis-1,3,5-naphtalènetrisulfonique.

4. Utilisation suivant la revendication 2, caractérisée en ce que, comme agent d'inhibition, on fait usage de l'un des composés disazoïques suivants : la benzidine, la dianilidurée, le pyridium, la néotropine, le rouge congo, le bleu de trypan, le rouge de trypan, le violet de trypan.

5. Produits susceptibles d'entrer localement en contact avec la peau, les muqueuses ou des sécrétions corporelles, caractérisés en ce qu'ils comprennent au moins un agent d'inhibition de la réverse-transcriptase des virus du groupe rétrovirus, en particulier de ceux responsables du sida, tels que HTLV I, HTLV II, HTLV III/LAV.

6. Produit suivant la revendication 5, caractérisé en ce qu'au moins un des agents d'inhibition est un composé disazoïque.

7. Produit suivant la revendication 6, caractérisé en ce qu'un agent d'inhibition est un sel hexasodique d'acide 8,8'-[carbonyl-bis-[imino-3,1 -phénylènecarbonylimino(4-méthyl-3,1 -phénylène)-carbonyl-imino]]-bis-1,3,5-naphtalènetrisulfonique.

8. Produit suivant la revendication 6, caractérisé en ce que, comme agent d'inhibition, il comprend l'un des composés disazoïques suivants la benzidine, la dianilidurée, le pyridium, la néotropine, le rouge congo, le bleu de trypan, le rouge de trypan, le violet de trypan.

9. Produit suivant l'une quelconque des revendications 5 à 8, à mettre en oeuvre comme composition ou produit cosmétique, hygiénique ou dentifrice.

10. Produit suivant l'une quelconque des revendications 5 à 8, à mettre en oeuvre comme composition ou produit de traitement thérapeutique quelconque, à application locale.

11. Produit suivant l'une quelconque des revendications 5 à 10, caractérisé en ce qu'il comprend une matière de support solide sur laquelle sont supportés par un procédé approprié quelconque le ou les agents d'inhibition.

12. Produit suivant la revendication 11, caractérisé en ce qu'il consiste en mouchoirs, serviettes, bandages, pansements, gants, produits d'emballage, préservatifs, diaphragmes, ouate, papier hygiénique et moyens analogues.

13. Produit suivant l'une quelconque des revendications 5 à 10, caractérisé en ce qu'il comprend une pâte, dans laquelle le ou les agents d'inhibition sont mélangés.

14. Produit suivant la revendication 13, caractérisé en ce qu'il consiste en une pâte dentifrice, un onguent, une crème, un shampoing, un fard.

15. Produit suivant l'une quelconque des revendications 5 à 10, caractérisé en ce qu'il comprend un véhicule liquide dans lequel le ou les agents d'inhibition sont en solution ou en suspension.

16. Produit suivant la revendication 15, caractérisé en ce qu'il consiste en un bain de bouche, une solution à pulvériser, un désodorisant, une lotion.

Office européen
des brevets

RAPPORT DE RECHERCHE EUROPEENNE

Numero de la demande

EP  88 87 0139

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | CHEMICAL ABSTRACTS, vol. 107, no. 21, 23 novembre 1987, page 22, résumé no. 190405j, Columbus, Ohio, US; K.D. JENTSCH et al.: "Inhibition of human immunodeficiency virus type I reverse transcriptase by suramin-related compounds", & J. GEN. VIROL. 1987, 68(8), 2183-92 <br> * Résumé * | 1-8 | A 61 K  31/655 |
| Y | CHEMICAL ABSTRACTS, vol. 104, no. 23, 9 juin 1986, page 42, résumé no. 199769b, Columbus, Ohio, US; J. BALZARINI et al.: "Comparative inhibitory effects of suramin and other selected compounds on the infectivity and replication of human T-cell lymphotropic virus (HTLV-III)/lymphadenopathy-associated virus (LAV)", & INT. J. CANCER 1986, 37(3), 451-7 <br> * Résumé * | 1-8 | |
| E | FR-A-2 612 515  (ARGUENON) <br> * En entier; en particulier page 4, lignes 1-4,9-10 * | 1-8 | **DOMAINES TECHNIQUES RECHERCHES (Int. Cl.4)** <br><br> A 61 K |

Le présent rapport a été établi pour toutes les revendications

| Lieu de la recherche | Date d'achèvement de la recherche | Examinateur |
|---|---|---|
| LA HAYE | 31-03-1989 | SCARPONI U. |